# EUROPEAN PATENT SPECIFICATION

(11) **EP 1 664 732 B1**
(45) Date of publication and mention of the grant of the patent: **21.05.2008**
(21) Application number: 04762864.9
(22) Date of filing: 23.09.2004
(51) Int. Cl.: G01N 11/14, G01N 33/38

(54) **A METHOD OF MEASURING THE CONSISTENCY OF A MIXTURE AS WELL AS AN APPARATUS FOR CARRYING OUT THE METHOD**
VERFAHREN ZUR MESSUNG DER KONSISTENZ EINES GEMISCHS SOWIE VORRICHTUNG ZUR DURCHFÜHRUNG DES VERFAHRENS
PROCEDE DE MESURE DE LA CONSISTANCE D'UN MELANGE ET APPAREIL DE MISE EN APPLICATION DU PROCEDE

(30) Priority: 24.09.2003 DK 200301388
(43) Date of publication of application: 07.06.2006
(73) Proprietor: Convi, 5270 Odense (DK)
(72) Inventor: NIELSEN, Niels, H ll, DK-5270 Odense N (DK)
(74) Representative: Thierry-Carstensen, Ole Jean
(86) International application number: PCT/DK2004/000644
(87) International publication number: WO 2005/029045

(56) References cited:
- WO-A-03/048743
- US-A- 4 299 119
- US-A- 5 321 974
- US-A- 5 456 105
- US-B1- 6 227 039

## Description

### Technical Field

The invention relates to a method of measuring the consistency of a mixture during a mixing process, said method involving a measuring of the force exerted on a measuring device immersed in the mixture while said mixture is subjected to a movement relative to said measuring device by means of an agitator of a mixer. The invention relates furthermore to an apparatus for carrying out the method.

### Background Art

During the production of concrete, the flowability, viz. the workability of the concrete is controlled by way of two fundamentally different methods. One method is based on a measurement of the power consumed by the mixer as an expression of how viscous the concrete is. In many respects this method has been developed so as to correct various weaknesses, but the method suffers inter alia from the weakness that the modem concrete mixtures are so fluid that nothing but an insignificant difference applies to the power consumed by the mixer when said mixer is idle running and when the mixing of the ready concrete takes place.

The second method is based on a measuring of the moisture present in the materials involved, either before or after said materials have been placed in the mixer, whereafter the amount of water to be added in order to obtain the desired flowability is calculated.

Both methods are focussed on developing a recipe for the desired concrete whereafter the data of the recipe are registered. These methods do not result in a measurement of the viscosity of the concrete, but merely in a comparison value based on a single parameter, viz. either on the power consumed by the mixer or on the present amount of water, and such a single parameter is not providing a sufficient description of the flowability of the concrete.

Rheology teaches the flowing properties of various substances. The flowing properties of a substance can be mathematically expressed by the expression y = ax + b, i.e. the equation of a straight line. In the equation y indicates a power and x indicates a speed. In order to describe the flowability of a substance, the two constants a and b must be determined, which can be done in viscosimeters adapted to such a purpose and determining the shear stress in the substance in question at two different speeds.

The above measurement of the power consumed by the mixer results in nothing but a determination of a point on the straight line, which does not suffice for defining said line. In addition, the registration of the moisture does not involve a measuring of the flowability of the concrete at all, but in stead a measuring of the water content. The water content represents only one factor of the many factors affecting the flowing properties. The amount of water and the amount of plasticizing additives do indeed represent the two factors with the highest effect on the flowing properties. However, factors, such as the grain size distributions and the grain shape of the individual aggregate fractions, the hardness of the water and the reaction of the additives in the relevant mixture as well as the temperature of the concrete and the variations of the cement and the fly ash have also a significant effect on the flowing properties when it is a question of a substance, such as concrete.

Viscosimeters are known for measuring the flowing properties of concrete, but the use thereof is not advantageous because the measuring is carried out outside the mixer. Such a measuring requires many resources, and when the measurement forms the basis of an approval of the concrete, said measuring prolongs the cycle time of a mixture. The use of a viscosimeter renders it possible to determine the above straight line. By a known viscosimeter, the concrete mixture is poured into a container in which measuring devices are immersed. Subsequently, the container is caused to rotate, and the force exerted by the concrete on the measuring device is measured. The force measured depends on the rotating speed of the container and on the rheological properties of the concrete. The yield stress can be described as the force necessary for causing the concrete to move and corresponds to the above constant b, and the viscosity is an expression of the force necessary for increasing the rotational speed. The latter force corresponds to the above factor a, where the speed representing the variable x is the rotational speed at the measuring site.

### Disclosure of Invention

The object of the invention is to provide a method and an apparatus rendering it possible to determine the flowing properties of a mixture, especially of a concrete mixture, directly in connection with the mixing process per se.

This object is according to the invention achieved by carrying out the measuring of the exerted force and the speed at at least two different relative speeds inside the mixer during the movement of the agitator.

As a result the flowing properties of the mixture in question can be determined without delaying the mixing process. In addition, based on known relationships between the effect of the various ingredients and of the variations in said ingredients on the factors a and b in the above formula, the measuring results can be used for determining the parameters to be adjusted for obtaining the desired flowing properties.

According to the invention, the speeds can be determined as the maximum speed and the minimum speed at the measuring site in question based on the geometry and rotational speed of the agitator, and the corresponding exerted forces are registered when the measurements thereof are at a maximum and minimum, respectively. Based on the knowledge that in a modem mixer the rotational speed of the driving shaft and the relative speeds of the agitator are substantially unaffected by the flowing properties of the mixture in question, it is possible in advance to determine the maximum speed and the minimum speed at the measuring site in question. The Based on the knowledge that in a modem mixer the rotational speed of the driving shaft and the relative speeds of the agitator are substantially unaffected by the flowing properties of the mixture in question, it is possible in advance to determine the maximum speed and the minimum speed at the measuring site in question. The measurements relating to the mixing are thus restricted to a measurement of the forces exerted at the measuring site in question while the mixer is running, and thus the exerted forces corresponding to the maximum speed and the minimum speed are only registered when said speeds are at a maximum and minimum, respectively.

Based on these two measurements, two points are determined on the line corresponding to the above equation.

The apparatus for carrying out the method is according to the invention advantageously a mixer with an agitator, where at least one force measuring device is associated with the agitator.

When the agitator according to the invention includes parts moving at varying speeds relative to the mixture during the mixing process while being in contact with said mixture, the force measuring device may according to the invention advantageously be provided in connection with at least one of said parts in such a manner that the speeds can be calculated in advance.

### Brief Description of the Drawings

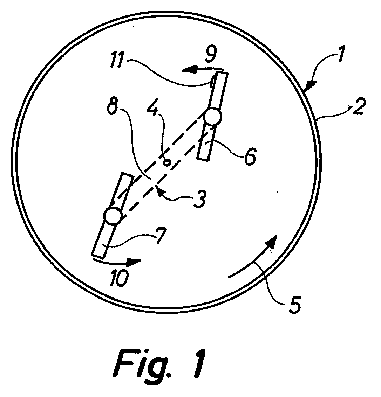

The invention is explained in detail below with reference to the accompanying drawings, in which
Fig. 1 is a diagrammatic top view of an apparatus according to the invention, and
Fig. 2 is a graphical view of measurements carried out in accordance with the present invention.

### Best Mode for Carrying out the Invention

The mixer illustrated in the drawing is designated the general reference numeral 1 and includes a circumferential wall 2 and an agitator designated the general reference numeral 3. The agitator is of the planetary mixer type, i.e. it includes a main driving shaft 4 causing the agitator to rotate in the direction of an arrow 5 and carrying two agitating members 6 and 7. These agitating members 6 and 7 are carried at each end of a substantially horizontal carrying device 8. The agitating members 6 and 7 are caused to rotate individually in the direction of the arrows 9 and 10, respectively. A measuring device 11 is arranged on one of these two agitating members 6 and 7. This measuring device 11 is placed at a location being exerted to a force during the mixing process.

When the mixer is running, the agitator is caused to rotate by means of the driving shaft 4 at a specific rotational speed, and the simultaneous rotation of the agitating members 6 and 7 per se has the result that the relative movement between the measuring site in question and the mixture in the mixer 1 varies as a function of the position of the agitating member 6 relative to the carrying device 8.

The force exerted at the measuring device 11 is suitably provided by means of a strain gauge, and the resulting measurement is transferred to a receiving station (not shown) via a radio signal. The latter signal is subsequently transferred to a control device (not shown) where it is subjected to a processing. The energy necessary for this purpose can be provided in a conventionally known manner by means of for instance batteries, and the necessary components are mounted in a suitably protected manner on the parts in question of the agitator 3.

When the mixer according to the invention is used, the speed of the measuring device 11 is initially calculated relative to the concrete mixture in question on the basis of the rotational speed of the agitator 3 and the rotational speed of the agitating members 6 and 7 as well as on the basis of the position of said measuring device 11. During the mixing process, a continuous measuring is carried out at regular intervals of the force exerted on said measuring device 11, and based on these measurements the registration of maximum and minimum force are deducted. The measuring result is used by the control device in question for determining a line defined by the equation y = ax + b, which represents the flowing properties of the mixture in question. Two points on the line are determined by means of on one hand the measured maximum force and the calculated maximum speed and on the other hand the measured minimum force and the calculated minimum speed. Based on the knowledge of the effect of the various components in the mixture on the constants a and b in the equation, it is subsequently possible to add further additives in such a manner that a mixture possessing the desired flowing properties is obtained, i.e. the desired yield stress and the desired viscosity.

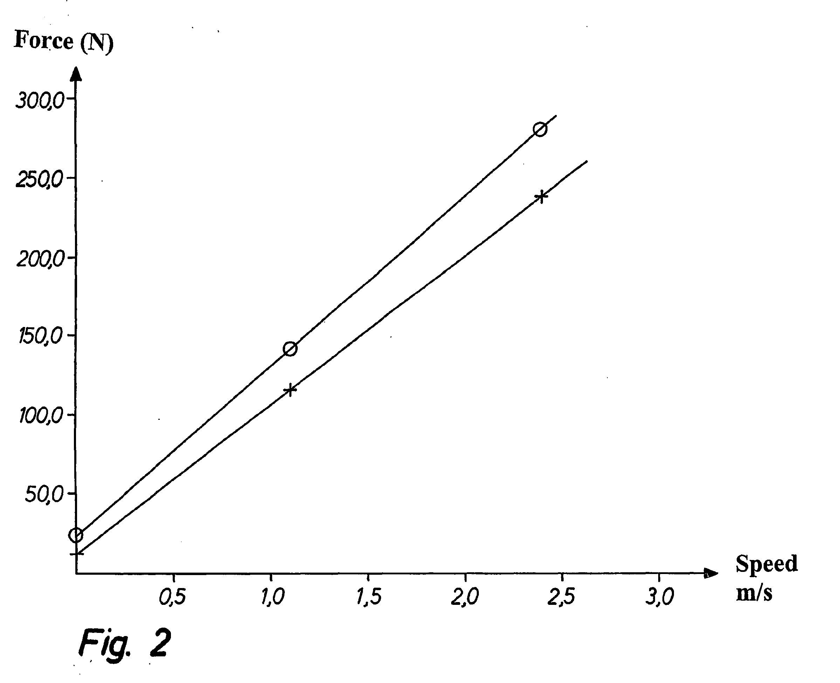

Fig. 2 illustrates an example of the use of the invention. Fig. 2 has been drafted on the basis of the following measurements carried out in a mixture after 100 seconds and 130 seconds, respectively, from the activation of a mixer. At 110 seconds, 7 litres of water per m³ of concrete were added on the basis of the measurements carried out at 100 seconds. The measurements carried out and the subsequent calculations appear from the following table:

| | 100 sec. | 130 sec. | Speed |
|---|---|---|---|
| Min | 139 | 115 | 1.1 |
| Max | 276 | 236 | 2.4 |
| a (calculated) | 105.4 | 93.1 | |
| b (calculated) | 23.1 | 12.6 | |

Measurings carried out after 100 seconds appear at the symbol o and after 130 seconds at the symbol +.

The invention has been described with reference to a preferred embodiment. Many modifications can be carried out without thereby deviating from the scope of the invention. Separate probes can for instance be mounted in connection with the agitator for measuring the exerted forces and for instance such that two probes are arranged at their respective distances from the axis of rotation of the driving shaft with the result that measurings of the force are carried out at various speeds.

## Claims

1. A method of measuring the consistency of a mixture during a mixing process using an agitator of a planetary type comprising a rotatable agitator (3) carrying individually rotating agitating members (6, 7), **characterised by** involving a measuring of the force exerted on a measuring device (11) arranged on one of the individually rotating agitating members (6, 7) and immersed in the mixture while said mixture is subjected to the mixing process, said agitator (3) being driven at a specific rotational speed during the mixing, whereby the measuring of the exerted force and a calculation of the corresponding speed is carried out at at least two different relative positions of the agitating member carrying the measuring device (11) during the movement of the agitator, and the determination of the flowing properties is based on the values of force and speed.

2. A Method according to claim 1, **characterised by** the speeds being determined as the maximum speed and the minimum speed at the measuring site in question based on the geometry and rotational speed of the agitator (3), and by the corresponding exerted forces being registered when the measurements thereof arc at a maximum and minimum, respectively.

3. An apparatus for carrying out the method according to claims 1 to 2, and including a mixer (1) of a planetary type comprising a rotatable agitator (3) carrying individually rotating agitating members (6, 7), **characterised in that** at least one force measuring device (11) is arranged on one of the individually rotating agitating members (6, 7), such that in use it is immersed in the mixture.

4. An apparatus according to claim 3, and where the agitator (3) includes members (6, 7), moving at a varying speed relative to the mixture during the mixing process while being in contact with said mixture, **characterised in that** the force measuring device (11) is provided in connection with at least one of said members (6, 7).

## Patentansprüche

1. Verfahren zur Messung der Konsistenz eines Gemischs während eines Mischverfahrens unter Nutzung eines Rührwerks eines Planetentyps, umfassend ein drehbares Rührwerk (3), das einzeln rotierende Rührwerkelemente (6, 7) trägt, **gekennzeichnet durch** die Einbeziehung einer Messung der Kraft, die auf eine Messvorrichtung (11) aufgebracht wird, die auf einem der einzeln rotierenden Rührwerkelemente (6, 7) angeordnet ist und in das Gemisch eingetaucht wird, während das Gemisch dem Mischverfahren unterzogen wird, wobei das Rührwerk (3) während des Mischens mit einer spezifischen Drchgeschwindigkeit angetrieben wird, wodurch das Messen der aufgebrachten Kraft und eine Berechnung der entsprechenden Geschwindigkeit an zumindest zwei unterschiedlichen relativen Positionen des Rührwerkelements, welches die Messvorrichtung (11) trägt, während der Bewegung des Rührwerks vorgenommen wird, und die Bestimmung der Fließeigenschaften auf den Werten von Kraft und Geschwindigkeit beruht.

2. Verfahren nach Anspruch 1, **dadurch gekennzeichnet, dass** die Geschwindigkeiten als die maximale Geschwindigkeit und die minimale Geschwindigkeit an der betreffenden Mcssstelle auf der Grundlage der Geometrie und der Drehgeschwindigkeit des Rührwerks (3) bestimmt werden, und dass die entsprechenden ausgeübten Kräfte registriert werden, wenn die Messungen derselben bei einem Maximum bzw. einem Minimum vorgenommen werden.

3. Vorrichtung für die Durchführung des Verfahrens nach den Ansprüchen 1 bis 2, einschließlich eines Mischers (1) eines Planetentyps, umfassend ein drehbares Rührwerk (3), das einzeln rotierende Rührwerkelemente (6, 7) trägt, **dadurch gekennzeichnet, dass** zumindest eine Kraftmessvorrichtung (11) auf einem der einzeln rotierenden Rührwerkelemente so angeordnet ist, dass sie beim Einsatz in das Gemisch eingetaucht wird.

4. Vorrichtung nach Anspruch 3, wobei das Rührwerk (3) Elemente (6, 7) einschließt, die sich bei einer sich ändernden Geschwindigkeit relativ zum Gemisch während des Mischverfahrens bewegen, während sie in Kontakt mit dem Gemisch sind, **dadurch gekennzeichnet, dass** die Kraftmessvorrichtung (11) in Verbindung mit zumindest einem der Elemente (6, 7) bereitgestellt wird.

## Revendications

1. Procédé de mesure de la consistance d'un mélange pendant un processus de mélange en utilisant un agitateur d'un type planétaire comprenant un agitateur rotatif (3) portant des éléments d'agitation (6, 7) individuellement rotatifs, **caractérisé en ce que** cela implique une mesure de la force exercée sur un dispositif de mesure (11) agencé sur un des éléments agitateurs (6, 7) individuellement rotatifs et immergé dans le mélange tandis que ledit mélange est soumis au processus de mélange, ledit agitateur (3) étant entraîné à une vitesse de rotation spécifique pendant le mélange, la mesure de la force exercée et un calcul de la vitesse correspondante étant réalisés dans au moins deux différentes positions relatives de l'élément d'agitation portant le dispositif de mesure (11) pendant le déplacement de l'agitateur, et la détermination des propriétés d'écoulement est basée sur les valeurs de la force et de la vitesse.

2. Procédé selon la revendication 1, **caractérisé en ce que** les vitesses sont déterminées en tant que vitesse maximale et vitesse minimale au niveau du site de mesure en question en se basant sur la géométrie et la vitesse de rotation de l'agitateur (3), et par les forces correspondantes exercées enregistrées lorsque ses mesures se trouvent à un maximum et à un minimum, respectivement.

3. Appareil de réalisation du procédé selon l'une quelconque des revendications 1 à 2, et comportant un mélangeur (1) d'un type planétaire comprenant un agitateur rotatif (3) portant des éléments d'agitation (6, 7) individuellement rotatifs, **caractérisé en ce qu'**au moins un dispositif de mesure de force (11) est agencé sur l'un des éléments d'agitation (6, 7) individuellement rotatifs, de telle sorte qu'au cours d'une utilisation il est immergé dans le mélange.

4. Appareil selon la revendication 3, et dans lequel l'agitateur (3) comporte des éléments (6, 7), se déplaçant à une vitesse variable par rapport au mélange pendant le processus de mélange tout en étant en contact avec ledit mélange, **caractérisé en ce que** le dispositif de mesure de force (11) est prévu en connexion avec au moins un desdits éléments (6, 7).
